# EUROPEAN PATENT SPECIFICATION

(11) **EP 2 840 913 B1**
(45) Date of publication and mention of the grant of the patent: **14.02.2018**
(21) Application number: 12875611.1
(22) Date of filing: 26.04.2012
(51) Int. Cl.: A24F 47/00

(54) **ELECTRONIC CIGARETTE WITH SEALED CARTRIDGE**
ELEKTRONISCHE ZIGARETTE MIT VERSIEGELTER KARTUSCHE
CIGARETTE ÉLECTRONIQUE AYANT UNE CARTOUCHE SCELLÉE

(43) Date of publication of application: 04.03.2015
(73) Proprietor: Fontem Holdings 1 B.V., 1083 HN Amsterdam (NL)
(72) Inventor: HON, Lik, Hong Kong (CN)
(74) Representative: Gulde & Partner
(86) International application number: PCT/CN2012/000562
(87) International publication number: WO 2013/159245

(56) References cited:
- WO-A1-2010/145805
- CN-U- 202 052 669
- CN-U- 202 052 669
- CN-U- 202 197 836
- CN-U- 202 197 836
- DE-A1-102007 011 120
- DE-A1-102007 011 120

## Description

### BACKGROUND OF THE INVENTION

Electronic cigarettes or vapor inhalers generally use a heater to vaporize liquid nicotine, or other liquid substances. The user inhales on the electronic cigarette drawing ambient air through the electronic cigarette housing. The vapor or mist mixes with the air flow moving through the housing and is inhaled by the user.

In comparison to real tobacco cigarettes, electronic cigarettes have many advantages. The risks of lung cancer associated with real tobacco cigarettes is largely avoided with electronic cigarettes because the tar and other chemicals in tobacco linked to lung cancer are not present in an electronic cigarette. Electronic cigarettes generate vapor or mist, and not smoke. Consequently, there is no comparable second-hand smoke problem with use of electronic cigarettes. In addition, since there is no burning material in electronic cigarettes, the risk of fire is eliminated.

The liquid in electronic cigarettes is stored in a bottle or in absorbent material within the housing. During storage and handling, the liquid may leak due to vibration, temperature variations, and other factors. The liquid may also be negatively affected by exposure to the environment. Accordingly, there is a need for an improved electronic cigarette. CN 202 052 669 discloses an electronic cigarette with a piercing element to pierce a diaphragm of a liquid storage element.

### SUMMARY OF THE INVENTION

In a new electronic cigarette, separate cartridge and vaporizer units are provided. The cartridge unit has a cartridge tube containing a liquid with a seal sealing the liquid within the cartridge tube. The vaporizer unit may have a piercer and a heater, with the front side of the vaporizer unit moveable into engagement with the cartridge unit, causing the piercer to pierce the seal in preparation for use of the electronic cigarette. A battery may be connected to a back side of the vaporizer unit via a connector. The vaporizer unit may also have and an electronic circuit electrically connected to the heater and to an inhalation sensor, or a switch activated by the user.

Further provided is a cartridge unit for use with an electronic cigarette, comprising an overtube including an outlet at a front end of the overtube; a cartridge tube within the overtube, the cartridge tube having a closed front end and an open back end; a liquid saturated material within the cartridge tube; a seal at the back end of the cartridge tube sealing the liquid saturated material within the cartridge tube; and at least one air flow pathway between the overtube and the cartridge tube.

The material may comprise fibers saturated with a nicotine solution.

Further provided is a vaporizing unit for use in an electronic cigarette, comprising an arm base; two or more arms on a front side of the base; a heater between the arms; and a piercer between the arms extending forward of the heater.

The vaporizing unit may further include a circuit board on the base electrically linked to the heater.

The vaporizing unit may further include a hole in the circuit board aligned with the heater, and further including a flow sensor positioned to sense air flow past the heater.

The vaporizing unit may further comprise a socket on a back side of the base adapted to connect with a battery. Optionally, a fiber heater tube may be supported on the piercer, with the heater surrounding the heater tube, or a chamber tube for trapping liquid, extending into a ring chamber in the arm base, may also be provided.

### BRIEF DESCRIPTION OF THE DRAWINGS

In the drawings, the same reference number indicates the same element in each of the views.
Fig. 1. is a perspective view of a new electronic cigarette.
Fig. 2 is a section view of the electronic cigarette shown in Fig. 1.
Fig. 3 is a perspective section view of the electronic cigarette shown in Fig. 1.
Fig. 4 is an enlarged perspective view of the vaporizer assembly shown in Figs. 1-3.
Fig. 5 is an enlarged perspective view of the vaporizer plate shown in Fig. 4.
Fig. 6 is an enlarged perspective view of the arm base shown in Figs. 1 and 3.
Fig. 7 in a perspective view of the cartridge tube shown in Figs. 1-3.
Fig. 8 is a perspective section view showing the arm base of Fig. 6 positioned in the over tube of Figs. 1-3, in a ready-to-use position.

### DETAILED DESCRIPTION

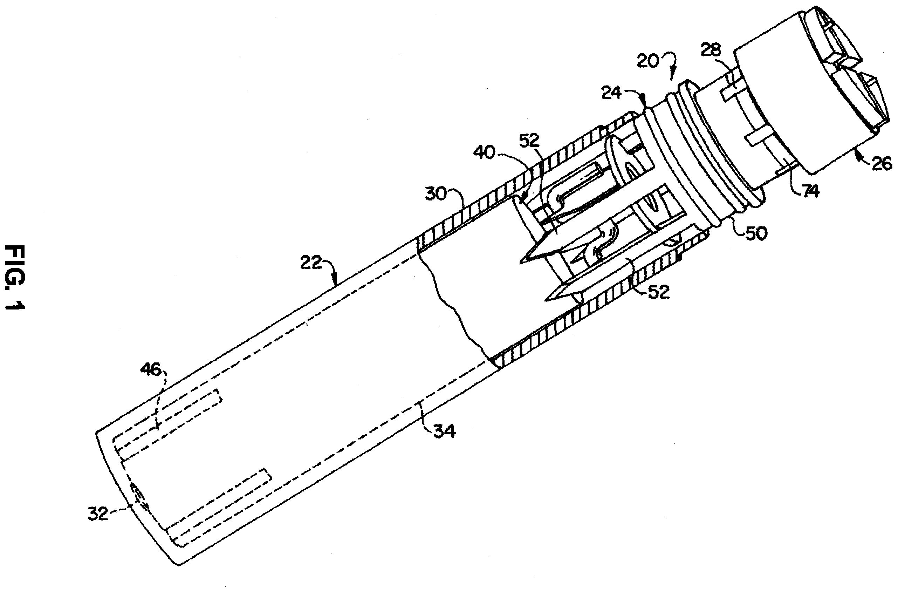

As shown in Fig. 1 an electronic cigarette 20 has a cartridge unit 22, a vaporizing unit 24 and a connector 26. As shown in Figs. 1-3, the cartridge unit 22 includes an overtube 30 having an outlet 32 at the front end. A cartridge tube 34 within the overtube 30 has a closed front end and an open back end 40. Fiber material 36 containing a liquid, such as liquid nicotine, is sealed within the cartridge tube 34 by a foil or membrane seal 38 at the back end of the cartridge tube 34. The closed front end of the cartridge tube may be spaced apart from the front wall of the overtube 30 by standoffs 46.

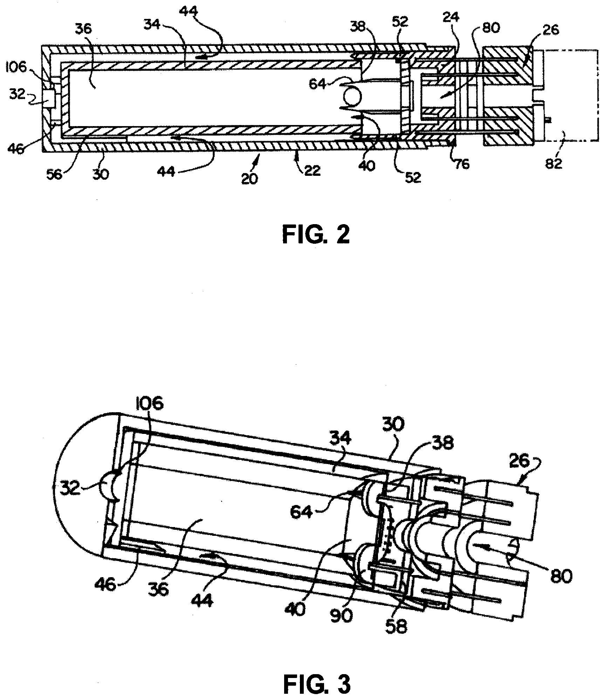

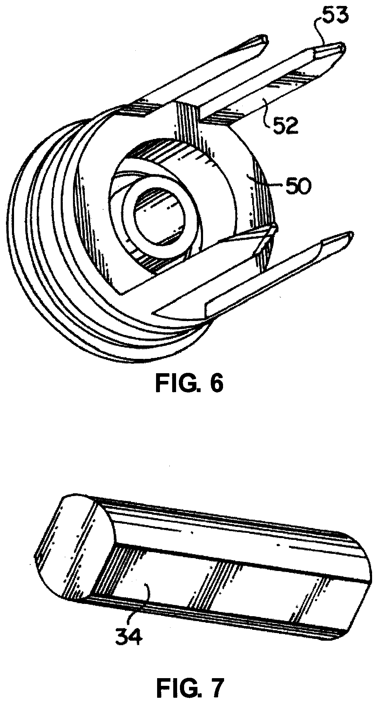

As shown in Figs. 2 and 3, a flow path 44 is formed between the cartridge tube 34 and the over tube 30. As shown in Fig. 7, the cartridge tube may have flat sides so that crescent shaped flow paths are formed between the cartridge tube 34 and the over tube 34 when the cartridge tube is inserted into the over tube. Alternatively, various other cartridge tube shapes may be used to provide one or more flow paths between the cartridge tube and the over tube. Although the Figures show the cartridge tube 34 centered within the overtube 30, in some designs, it may also be offset to one side. Inserts or baffles may also be used inside the overtube to create various other flow path shapes and configurations.

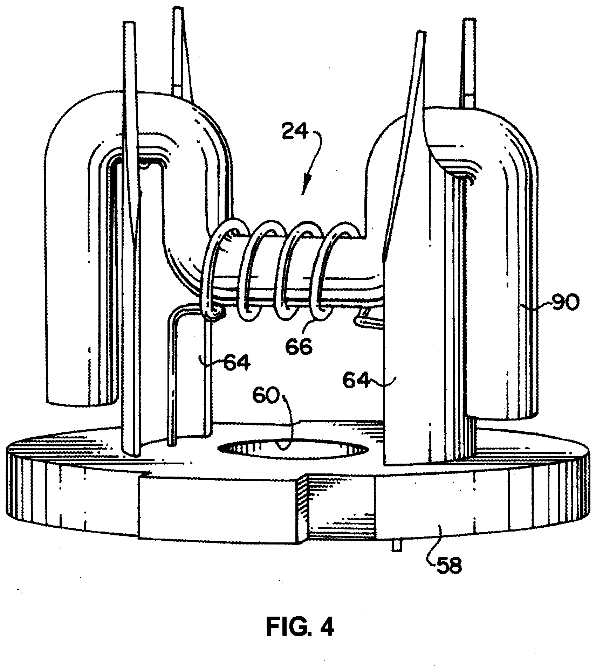

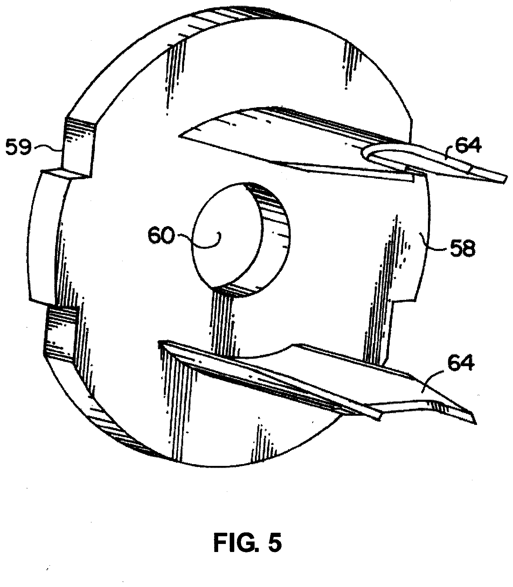

Turning to Figs. 4 and 5, the vaporizing unit 24 may have one or more piercing blades 64 attached to a plate 58 having a through hole 60. A heater tube 90 may be supported on cutouts in the blades 64. The heater tube may be made of quartz fiber, or other high temperature resistant wicking or absorbent material. A central section of the heater tube 90 extends within a heater coil 66. As shown in Figs. 1-3, 6 and 8, arms 52 are attached to the front side of an arm base 50, with socket slots 54 on the back side of the arm base 50. The tips of the arms are tapered into wedges 53. The design shown has four arms, although more or less arms may be provided as well. With the plate 58 assembled onto the arm base 50, the arms 52 may lie in recesses 59 in the plate 58, with the through hole 60 in the plate aligned with a corresponding through hole in the arm base 50. The wire lead of the heating coil 66 may connect to a circuit board or electronic chip directly or indirectly. It is also possible to connect the ends of the heating coil 66 to the blades 64, and with the blades electrically connected to the circuit board or electronic chip, which may be included in or on the plate 58, or located at another position within electronic cigarette 20.

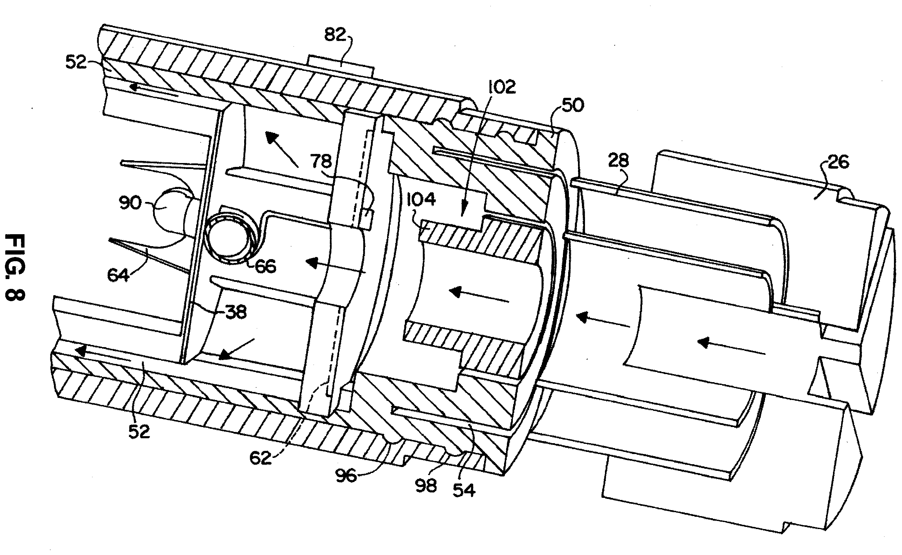

As shown in Figs. 1 and 8, the connector 26 may have rings 28 that can plug into the slots 54 on the back side of the arm base 50. Alternatively, the connector 26 may screw or snap onto the vaporizing unit 24. The back end of the connector 26 is adapted to receive or plug into a battery 82.

As shown in Fig. 8, the arm base 50 has first and second spaced apart detent rings 96 and 98 adapted to snap into first and second semicircular grooves at the back end of the arm base. In the first detent position, the detent ring 96 is in the outer groove, closest to the back end of the arm base 50. In this position, the blades 64 are held away from the seal 38 on the cartridge tube 34. The electronic cigarette 20 is provided from the factory in this position. Consequently, the cartridge tube 34 remains sealed during shipping and storage. This avoids any leaking or evaporation of liquid, for example nicotine solution, from the cartridge tube during shipping and storage. In addition, since the detent ring 96 firmly holds the arm base in position on the over tube 30, the seal 38 remains intact even with the vibration and shock impulses that typically occur during shipping and handling.

Turning once again to Figs. 1-3 and 8, when ready for use, the arm base 50 is pushed forward into the over tube 30, causing it to move into the position shown in Fig, 8. During this movement, the first detent ring 96 moves from the outer groove into the inner groove. The optional second detent ring 98, previously outside of the over tube 30, correspondingly moves into the outer groove. This longitudinal movement of the arm base 50 drives the front end of vaporizing unit 24 through the seal 38, with the heater tube 90 moving into the cartridge tube 34, as shown in Fig. 8. The spacing between the outer groove and the inner groove consequently determine the depth of insertion of the heater tube 90 into the fiber core 36 of the cartridge tube 34.

When the arm base 50 is pushed forward into the over tube 30, as described above, the wedge ends 53 on the arms move between the cartridge tube 34 and the inner walls of the over tube 30, to firmly hold the cartridge tube in place. At the same time, the blades 64 pierce and cut through the seal 38 and the heater tube 90 moves by the pre-set distance into the cartridge tube. As shown in Fig. 8. the heater tube 90 is then in direct contact with the liquid filled or liquid impregnated core 36 of the cartridge tube 34. Liquid from the core 36 wicks or otherwise moves into or onto the heater tube 90.

As shown in Fig. 8, the arm base 50 may include an annular ring chamber 102 around a chamber tube 104. After the seal is pierced, any liquid leaking out of the cartridge tube may collect in the ring chamber 102, rather than travel back through the chamber tube 104 to the battery. Similarly, as shown in Fig. 2, an annular liquid barrier 106 may be provided around the inlet 32 on the inner wall of the front end of the over tube 30, to help prevent any liquid from moving out of the inlet 32.

A battery is attached onto the connector 26, either before or after the forward piercing movement. The battery is electrically connected to the circuit board 62 via the metal rings on the connector 26 plugging into the sockets 54 having leads to the circuit board 62.

The user inhales on the front end of the overtube 30, drawing air out of the outlet 32. Air flows through the continuous central opening in the connector 26 and in the vaporizing unit 24. A sensor 78 shown in Fig. 8, either on or electrically connected to the circuit board 62, may be used to detect air flow through the central passageway in the vaporizing unit 24. Upon detection of air flow, the circuit board 62 switches on electric current to the heater coil 66. Alternatively, as shown in Fig. 8, a manual switch 82 may be provided on base, with the user activating the switch to turn the heater on while the user is inhaling. Liquid from the cartridge tube 34 is vaporized and mixed with the flowing air. The mixture of vapor and/or mist and air moves forward through the flow path 44, through the outlet 32, and is inhaled by the user.

As shown in Fig. 3, there is no opening at the front end of the cartridge tube 34. Accordingly, air cannot flow through the cartridge tube. Rather, upon inhalation, as shown by the arrows in Fig. 8, air flows through the vaporizing unit 24, but not through the cartridge tube 34. This makes the electronic cigarette 20 much more resistant to liquid leaking resulting from blowing into the outlet. In many existing designs, if the user blows into the outlet, instead of inhaling, liquid leaking may increase dramatically. With the present design, however, blowing into the outlet does not cause any great increase in leaking, because it does not create any positive pressure acting to push liquid out of the cartridge tube.

The cartridge tube 34 may be filled with a liquid saturated fiber or sponge-like or porous material, or another material capable of holding liquid. The arm base 50 and the electrical contacts 74 on the back of the arm base may be copper or other electrically conductive metal. The arm base 50 and may also be made of plastic or ceramic, with separate wire leads provided to make electrical connections. The blades 64 or other piercer may be metal, such as stainless steel, another metal, or a non-metal material such as plastic. A single blade or pointed piercer may be used. Optionally, the blades or piercer may be replaced by a projection on the heater tube 90, if used, or on the heater 66.

The blades 64 may optionally be attached to the arms 52, instead of the plate 58. The heater coil 66 may be a coil of wire, or other form of electrical heater, including heater wires in non-coil configurations. The seal 38 may be a metal foil seal, or a seal of other material, such as plastic. The circuit board 62 may be replaced with an electronic chip or component package.

The cartridge unit 22, the vaporizing unit 24 and the connector 26 may be provided as separate units, or together in an un-assembled kit. With the cartridge unit 22 separate from the vaporizing unit 24, the seal 38 is intact and seals the liquid within the cartridge tube 34. This avoids leaking during storage and handling. The seal also seals the liquid from the environment, which may increase the storage life of the liquid. The vaporizing unit 24 is designed for repeated use, while the cartridge unit 22 is a consumable single use unit. By providing the cartridge unit 22 and the vaporizing unit 24 as separate units, the user can reuse the vaporizing unit 24 over and over again, while replacing the cartridge unit 22 as needed. The electronic cigarette 20 can therefore be provided at lower cost.

Thus, novel designs have been shown and described. Various changes and substitutions may of course be made as defined by the following claims.

## Claims

1. An electronic cigarette, comprising:
a cartridge unit (22) including cartridge tube (34) containing a liquid, and a seal (38) on the cartridge tube (34) sealing the liquid within the cartridge tube (34); and
a vaporizer unit (24) having a piercer and a heater, with the vaporizer unit (24) moveable into engagement with the cartridge unit (22), causing the piercer to pierce the seal (38),
**characterized in that**
the heater comprises a heater tube (90) and a heating coil (66), the heater tube (90) being supported by blades (64) with the heating coil (66) surrounding the heater tube (90).

2. The electronic cigarette of claim 1 further comprising a battery (82) connected to the vaporizer unit (24).

3. The electronic cigarette of claim 1 with the vaporizer unit (24) further including an electronic circuit electrically connected to the heating coil (66) and to an inhalation sensor.

4. The electronic cigarette of claim 1 with the heater tube (90) being a fiber heater tube.

5. The electronic cigarette of claim 1 with the vaporizer unit (24) including arms (52) attached to a base (50), with the arms (52) having wedging tips for engaging the cartridge unit (22).

6. The electronic cigarette of claim 1 with the vaporizer unit (24) having a base (58), with the piercer and the heating coil (66) on a front side of the base (58), and the connector (26) on the back side of the base (58), with the connector (26) adapted to connect with a battery (82).

7. The electronic cigarette of claim 1 with the cartridge tube (34) inside of an overtube (30) and a flow path formed between the cartridge tube (34) and the overtube (30), with the flow path leading to an outlet at a front end of the overtube (30).

8. The electronic cigarette of claim 1 further including a chamber tube (104) extending into a ring chamber in the arm base (50), for trapping liquid.

9. The electronic cigarette of claim 1 further comprising:
a vaporizer unit having a heating coil around a heater tube, and an electronic circuit electrically connected to the heating coil and to an inhalation sensor, with the vaporizer unit moveable from a first detent position wherein the heater tube is spaced apart from the seal, to a second detent position, wherein the heater tube is moved through the seal and into the cartridge unit; and
a connector attached to the vaporizer unit.

## Patentansprüche

1. Elektronische Zigarette, umfassend:
eine Kartuscheneinheit (22), die eine Kartuschenröhre (34), die eine Flüssigkeit enthält, und ein Siegel (38) an der Kartuschenröhre (34), das die Flüssigkeit innerhalb der Kartuschenröhre (34) einschließt, umfasst; und
eine Verdampfereinheit (24), die ein Aufstechelement und ein Heizelement aufweist, wobei die Verdampfereinheit (24) in Eingriff mit der Kartuscheneinheit (22) bringbar ist, was bewirkt, dass das Aufstechelement das Siegel (38) aufsticht,
**dadurch gekennzeichnet, dass**
das Heizelement eine Heizröhre (90) und eine Heizwendel (66) umfasst, wobei die Heizröhre (90) durch Flügel (64) gehalten wird, wobei die Heizwendel (66) die Heizröhre (90) umgibt.

2. Elektronische Zigarette nach Anspruch 1, die ferner eine Batterie (82) umfasst, die mit der Verdampfereinheit (24) verbunden ist.

3. Elektronische Zigarette nach Anspruch 1, wobei die Verdampfereinheit (24) ferner eine elektronische Schaltung umfasst, die mit der Heizwendel (66) und mit einem Inhalationssensor elektrisch verbunden ist.

4. Elektronische Zigarette nach Anspruch 1, wobei die Heizröhre (90) eine Faserheizröhre ist.

5. Elektronische Zigarette nach Anspruch 1, wobei die Verdampfereinheit (24) Arme (52) umfasst, die an einer Basis (50) angebracht sind, wobei die Arme (52) Klemmspitzen zum Ineingriffnehmen der Kartuscheneinheit (22) aufweisen.

6. Elektronische Zigarette nach Anspruch 1, wobei die Verdampfereinheit (24) eine Basis (58) aufweist, wobei das Aufstechelement und die Heizwendel (66) sich an einer Vorderseite der Basis (58) befinden und das Verbindungselement (26) sich an der Rückseite der Basis (58) befindet, wobei das Verbindungselement (26) dazu ausgelegt ist, mit einer Batterie (82) verbunden zu sein.

7. Elektronische Zigarette nach Anspruch 1, wobei die Kartuschenröhre (34) sich innerhalb einer Hüllröhre (30) befindet und zwischen der Kartuschenröhre (34) und der Hüllröhre (30) ein Strömungsweg ausgebildet ist, wobei der Strömungsweg zu einem Auslass an einem vorderen Ende der Hüllröhre (30) führt.

8. Elektronische Zigarette nach Anspruch 1, die ferner eine sich in eine Ringkammer in der Armbasis (50) hinein erstreckende Kammerröhre (104) zum Einfangen von Flüssigkeit umfasst.

9. Elektronische Zigarette nach Anspruch 1, ferner umfassend:
eine Verdampfereinheit, die eine Heizwendel um eine Heizröhre herum und eine elektronische Schaltung, die mit der Heizwendel und mit einem Inhalationssensor elektrisch verbunden ist, aufweist, wobei die Verdampfereinheit aus einer ersten Arretierungsposition, in der die Heizröhre von dem Siegel beabstandet ist, in eine zweite Arretierungsposition, in der die Heizröhre durch das Siegel hindurch und in die Kartuscheneinheit hinein bewegt wird, beweglich ist; und
ein Verbindungselement, das an der Verdampfereinheit angebracht ist.

## Revendications

1. Cigarette électronique, comprenant :
une unité de cartouche (22) comportant un tube de cartouche (34) contenant un liquide, et un élément d'étanchéité (38) sur le tube de cartouche (34) scellant le liquide à l'intérieur du tube de cartouche (34) ; et
une unité de vaporisation (24) comportant un poinçon et un élément chauffant, l'unité de vaporisation (24) étant mobile pour venir en prise avec l'unité de cartouche (22), amenant ainsi le poinçon à percer l'élément d'étanchéité (38),
**caractérisée en ce que**
l'élément chauffant comprend un tube d'élément chauffant (90) et une bobine chauffante (66), le tube d'élément chauffant (90) étant supporté par des lames (64) avec la bobine chauffante (66) entourant le tube d'élément chauffant (90).

2. Cigarette électronique selon la revendication 1, comprenant en outre une batterie (82) raccordée à l'unité de vaporisation (24).

3. Cigarette électronique selon la revendication 1, l'unité de vaporisation (24) comprenant en outre un circuit électronique raccordé électriquement à la bobine chauffante (66) et à un capteur d'inhalation.

4. Cigarette électronique selon la revendication 1, le tube d'élément chauffant (90) étant un tube d'élément chauffant en fibres.

5. Cigarette électronique selon la revendication 1, l'unité de vaporisation (24) comprenant des bras (52) fixés à une base (50), les bras (52) comportant des pointes en biseau pour venir en prise avec l'unité de cartouche (22).

6. Cigarette électronique selon la revendication 1, l'unité de vaporisation (24) comportant une base (58), le poinçon et la bobine chauffante (66) étant sur une face avant de la base (58), et le connecteur (26) sur la face arrière de la base (58), le connecteur (26) étant adapté pour le raccordement à une batterie (82).

7. Cigarette électronique selon la revendication 1, le tube de cartouche (34) étant à l'intérieur d'une enveloppe tubulaire (30) et un chemin d'écoulement étant formé entre le tube de cartouche (34) et l'enveloppe tubulaire (30), le chemin d'écoulement menant à une sortie sur une extrémité avant de l'enveloppe tubulaire (30).

8. Cigarette électronique selon la revendication 1, comprenant en outre un tube formant chambre (104) s'étendant dans une chambre annulaire dans la base des bras (50), pour piéger du liquide.

9. Cigarette électronique selon la revendication 1, comprenant en outre :
une unité de vaporisation comportant une bobine chauffante autour d'un tube d'élément chauffant, et un circuit électronique raccordé électriquement à la bobine chauffante et à un capteur d'inhalation, l'unité de vaporisation étant mobile d'une première position d'encliquetage dans laquelle le tube d'élément chauffant est espacé de l'élément d'étanchéité, à une seconde position d'encliquetage, dans laquelle le tube d'élément chauffant est déplacé à travers l'élément d'étanchéité et dans l'unité de cartouche ; et
un connecteur fixé à l'unité de vaporisation.
